# EUROPEAN PATENT SPECIFICATION

(11) **EP 1 542 974 B1**
(45) Date of publication and mention of the grant of the patent: **31.12.2008**
(21) Application number: 03797377.3
(22) Date of filing: 16.09.2003
(51) Int. Cl.: C07D 213/04, A61K 31/519, A61P 17/06, A61P 29/00

(54) **5-[((2,3-difluorophenyl)methyl)thio]-7-{[(1S,2S)-2-hydroxy-1-(hydroxymethyl)propyl]amino}thiazolo[4,5-d]pyrimidin-2(3H)-one as CXCR2 antagonist**
5-[((2,3-difluorophenyl)methyl)thio]-7-{[(1S,2S)-2-hydroxy-1-(hydroxymethyl)propyl]amino}thiazolo[4,5-d]pyrimidin-2(3H)-on als CXCR2 Antagonist
5-[((2,3-difluorophenyl)methyl)thio]-7-{[(1S,2S)-2-hydroxy-1-(hydroxymethyl)propyl]amino}thiazolo[4,5-d]pyrimidin-2(3H)-one en tant qu'antagoniste de CXCR2

(30) Priority: 20.09.2002 GB 0221829
(43) Date of publication of application: 22.06.2005
(73) Proprietor: AstraZeneca AB, 151 85 Södertälje (SE)
(72) Inventor: BROUGH, Stephen, John AstraZeneca R & D Charnwood, Loughborough, Leicestershire LE11 5RH (GB); MCINALLY, Thomas AstraZeneca R & D Charnwood, Loughborough, Leicestershire LE11 5RH (GB)
(74) Representative: Phillips, Neil Godfrey Alasdair
(86) International application number: PCT/GB2003/004000
(87) International publication number: WO 2004/026835

(56) References cited:
- WO-A-00/09511
- WO-A-01/25242

## Description

The present invention relates to a thiazolopyrimidinone compound, processes and intermediates used in its preparation, pharmaceutical compositions containing it and its use in therapy.

Chemokines play an important role in immune and inflammatory responses in various diseases and disorders, including asthma and allergic diseases, as well as autoimmune pathologies such as rheumatoid arthritis and atherosclerosis. These small secreted molecules are a growing superfamily of 8-14 kDa proteins characterised by a conserved four cysteine motif. At the present time, the chemokine superfamily comprises three groups exhibiting characteristic structural motifs, the Cys-X-Cys (C-X-C), Cys-Cys (C-C) and Cys-X₃-Cys (C-X₃-C) families. The C-X-C and C-C families have sequence similarity and are distinguished from one another on the basis of a single amino acid insertion between the NH-proximal pair of cysteine residues. The C-X₃-C family is distinguished from the other two families on the basis of having a triple amino acid insertion between the NH-proximal pair of cysteine residues.

The C-X-C chemokines include several potent chemoattractants and activators of neutrophils such as interleukin-8 (IL-8) and neutrophil-activating peptide 2 (NAP-2).

The C-C chemokines include potent chemoattractants of monocytes and lymphocytes but not neutrophils. Examples include human monocyte chemotactic proteins 1-3 (MCP-1, MCP-2 and MCP-3), RANTES (Regulated on Activation, Normal T Expressed and Secreted), eotaxin and the macrophage inflammatory proteins 1α and 1β (MIP-1α and MIP-1β).

The C-X₃-C chemokine (also known as fractalkine) is a potent chemoattractant and activator of microglia in the central nervous system (CNS) as well as of monocytes, T cells, NK cells and mast cells.

Studies have demonstrated that the actions of the chemokines are mediated by subfamilies of G protein-coupled receptors, among which are the receptors designated CCR1, CCR2, CCR2A, CCR2B, CCR3, CCR4, CCR5, CCR6, CCR7, CCR8, CCR9, CCR10 and CCR11 (for the C-C family); CXCR1, CXCR2, CXCR3, CXCR4 and CXCR5 (for the C-X-C family) and CX₃CR1 for the C-X₃-C family. These receptors represent good targets for drug development since agents which modulate these receptors would be useful in the treatment of disorders and diseases such as those mentioned above.

WO-01/25242 discloses a series of thiazolopyrimidinone compounds useful as CXCR2 antagonists. A compound within the scope of WO-01/25242, but not specifically disclosed therein, has now surprisingly been found to have an improved pharmacological profile when compared with the structurally most similar compounds from WO-01/25242 ie. Examples 2 and 7.

The present invention therefore provides a compound of formula (I)

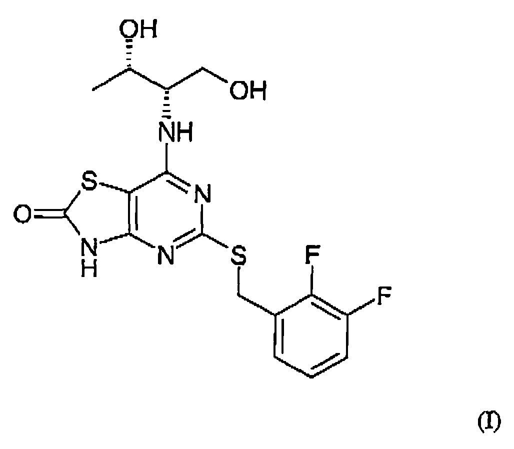

The compound of formula (I) is capable of existing in tautomeric form. Tautomers and mixtures thereof also form an aspect of the present invention,

According to the invention there is also provided a process for the preparation of compound (I) which comprises reaction of a compound of formula (II): 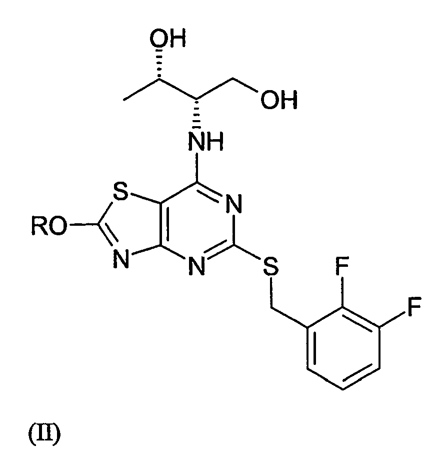 where R is C₁₋₆ alkyl with an acid,
and optionally thereafter:forming a pharmaceutically acceptable salt.

Preferably R is ethyl or methyl, more preferably methyl. Preferably the reaction is carried out using dioxan and HCl. Preferably the compounds of the invention are prepared according to the procedures exemplified herein.

The compound (II) can be prepared from the corresponding compound of formula (III): 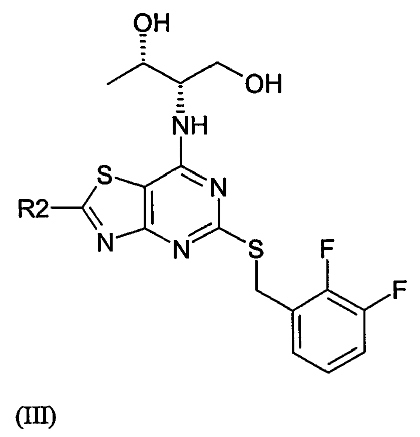 where R² is halogen by treating with a compound ROH in the presence of a base. Preferably the compound of formula (III) is treated with sodium methoxide. Preferably R² is chloro.

Compounds of formula (III) can be prepared using the sequence below and as described in WO-01/25242:

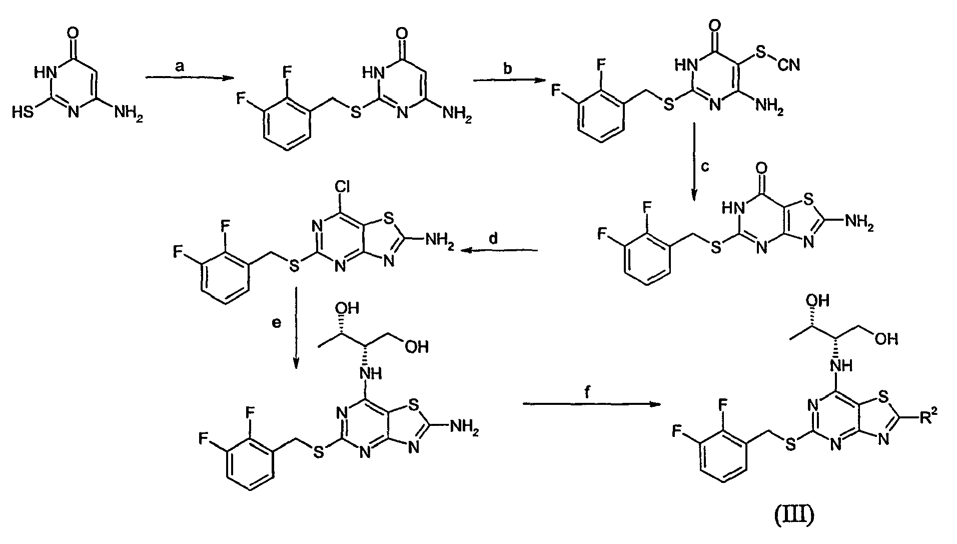

Suitable reagents for steps a to f will be known to those skilled in the art. Preferably steps a to f are carried out as exemplified herein.

The compound of formula (III) or (II) are also believed to be novel and forms a further aspect of the invention.

It will be appreciated by those skilled in the art that in the processes of the present invention certain functional groups such as hydroxyl or amino groups in the starting reagents or intermediate compound may need to be protected by protecting groups. Thus, the preparation of the compound of formula (I) may involve, at an appropriate stage, the removal of one or more protecting groups. The protection and deprotection of functional groups is fully described in 'Protective Groups in Organic Chemistry', edited by J. W. F. McOmie, Plenum Press (1973), and 'Protective Groups in Organic Synthesis', 2nd edition, T. W. Greene & P. G. M. Wuts, Wiley-Interscience (1991).

The compound of formula (I) above may be converted to a pharmaceutically acceptable salt or solvate thereof, preferably a basic addition salt such as sodium, potassium, calcium, aluminium, lithium, magnesium, zinc, benzathine, chloroprocaine, choline, diethanolamine, ethanolamine, ethyldiamine, meglumine, tromethamine or procaine, or an acid addition salt such as a hydrochloride, hydrobromide, phosphate, acetate, fumarate, maleate, tartrate, citrate, oxalate, methanesulphonate or *p*-toluenesulphonate.

The compound of formula (I) has activity as a pharmaceutical, in particular as a modulator of chemokine receptor (especially CXCR2) activity, and may be used in the treatment (therapeutic or prophylactic) of conditions/diseases in human and non-human animals which are exacerbated or caused by excessive or unregulated production of chemokines. Examples of such conditions/diseases include:
(1) **(the respiratory tract)** obstructive airways diseases including chronic obstructive pulmonary disease (COPD); asthma, such as bronchial, allergic, intrinsic, extrinsic and dust asthma, particularly chronic or inveterate asthma (e.g. late asthma and airways hyper-responsiveness); bronchitis; acute, allergic, atrophic rhinitis and chronic rhinitis including rhinitis caseosa, hypertrophic rhinitis, rhinitis purulenta, rhinitis sicca and rhinitis medicamentosa; membranous rhinitis including croupous, fibrinous and pseudomembranous rhinitis and scrofoulous rhinitis; seasonal rhinitis including rhinitis nervosa (hay fever) and vasomotor rhinitis; sarcoidosis, farmer's lung and related diseases, fibroid lung and idiopathic interstitial pneumonia;
(2) **(bone and joints)** rheumatoid arthritis, seronegative spondyloarthropathies (including ankylosing spondylitis, psoriatic arthritis and Reiter's disease), Behcet's disease, Sjogren's syndrome and systemic sclerosis;
(3) (skin) psoriasis, atopical dermatitis, contact dermatitis and other eczmatous dermitides, seborrhoetic dermatitis, Lichen planus, Pemphigus, bullous Pemphigus, Epidermolysis bullosa, urticaria, angiodermas, vasculitides, erythemas, cutaneous eosinophilias, uveitis, Alopecia areata and vernal conjunctivitis;
(4) **(gastrointestinal tract)** Coeliac disease, proctitis, eosinopilic gastro-enteritis, mastocytosis, Crohn's disease, ulcerative colitis, indeterminate colitis, microscopic colitis, inflammatory bowel disease, irritable bowel syndrome, non-inflammatory diarrhea, food-related allergies which have effects remote from the gut, e.g., migraine, rhinitis and eczema;
(5) **(central and peripheral nervous system)** Neurodegenerative diseases and dementia disorders, e.g. Alzheimer's disease, amyotrophic lateral sclerosis and other motor neuron diseases, Creutzfeldt-Jacob's disease and other prion diseases, HIV encephalopathy (AIDS dementia complex), Huntington's disease, frontotemporal dementia, Lewy body dementia and vascular dementia; polyneuropathies, e.g. Guillain-Barré syndrome, chronic inflammatory demyelinating polyradiculoneuropathy, multifocal motor neuropathy, plexopathies; CNS demyelination, e.g. multiple sclerosis, acute disseminated/haemorrhagic encephalomyelitis, and subacute sclerosing panencephalitis; neuromuscular disorders, e.g, myasthenia gravis and Lambert-Eaton syndrome; spinal disorders, e.g. tropical spastic paraparesis, and stiff-man syndrome: paraneoplastic syndromes, e.g. cerebellar degeneration and encephalomyelitis; CNS trauma; migraine; and stroke.
(6) **(other tissues and systemic disease)** Atherosclerosis, Acquired Immunodeficiency Syndrome (AIDS), lupus erythematosus, systemic lupus, erythematosus, Hashimoto's thyroiditis, type I diabetes, nephrotic syndrome, eosinophilia fascitis, hyper IgE syndrome, lepromatous leprosy, and idiopathic thrombocytopenia pupura; post-operative adhesions, and sepsis.
(7) Stroke, subarachnoid haemorrage, re-perfusion injury in the heart, brain, peripheral limbs and other organs
(8) (allograft rejection) acute and chronic following, for example, transplantation of kidney, heart, liver, lung, bone marrow, skin and cornea; and chronic graft versus host disease;
(9) Cancers, especially non-small cell lung cancer (NSCLC), malignant melanoma, prostate cancer and squamous sarcoma, and tumour metastasis;
(10) Diseases in which angiogenesis is associated with raised CXCR2 chemokine levels (e.g. NSCLC, diabetic retinopathy).
(11) Cystic fibrosis
(12) Bum wounds & chronic skin ulcers
(13) Reproductive Diseases (e.g. Disorders of ovulation, menstruation and implantation, Pre-term labour, Endometriosis)

Thus, the present invention provides a compound of formula (I), or a pharmaceutically-acceptable salt or solvate thereof, as hereinbefore defined for use in therapy.

Preferably the compound of the invention are used to treat diseases in which the chemokine receptor belongs to the CXC chemokine receptor subfamily, more preferably the target chemokine receptor is the CXCR2 receptor,

Particular conditions which can be treated with the compound of the invention are rheumatoid arthritis, diseases in which angiogenesis is associated with raised CXCR2 chemokine levels, and COPD. It is preferred that the compound of the invention is used to treat rheumatoid arthritis and respiratory disease.

The compound of formula (I) may have utility as an antagonist of the CX3CR1 receptor. Such a compound is expected to be particularly useful in the treatment of disorders within the central and peripheral nervous system and other conditions characterized by an activation of microglia and/or infiltration of leukocytes (e.g. stroke/ischemia and head trauma).

In a further aspect, the present invention provides the use of a compound of formula (I), or a pharmaceutically acceptable salt or solvate thereof, as hereinbefore defined in the manufacture of a medicament for use in therapy.

In a still further aspect, the present invention provides the use of a compound of formula (I), or a pharmaceutically acceptable salt or solvate thereof, as hereinbefore defined in the manufacture of a medicament for the treatment of human diseases or conditions in which modulation of chemokine receptor activity is beneficial.

In the context of the present specification, the term "therapy" also includes "prophylaxis" unless there are specific indications to the contrary, The terms "therapeutic" and "therapeutically" should be construed accordingly.

We further provide a method of treating a chemokine mediated disease wherein the chemokine binds to a chemokine (especially CXCR2) receptor, which comprises administering to a patient a therapeutically effective amount of a compound of formula (I), as hereinbefore defined.

The invention also provides a method of treating an inflammatory disease, especially rheumatoid arthritis, COPD, respiratory disease or psoriasis, in a patient suffering from, or at risk of, said disease, which comprises administering to the patient a therapeutically effective amount of a compound of formula (I), as hereinbefore defined.

For the above-mentioned therapeutic uses the dosage administered will, of course, vary with the compound employed, the mode of administration, the treatment desired and the disorder indicated.

The compound of formula (I) may be used on its own but will generally be administered in the form of a pharmaceutical composition in which the formula (I) compound (active ingredient) is in association with a pharmaceutically acceptable adjuvant, diluent or carrier. Depending on the mode of administration, the pharmaceutical composition will preferably comprise from 0.05 to 99 %w (per cent by weight), more preferably from 0.05 to 80 %w, still more preferably from 0.10 to 70 %w, and even more preferably from 0.10 to 50 %w, of active ingredient, all percentages by weight being based on total composition.

The present invention also provides a pharmaceutical composition comprising a compound of formula (I) as hereinbefore defined, in association with a pharmaceutically acceptable adjuvant, diluent or carrier.

The invention further provides a process for the preparation of a pharmaceutical composition of the invention which comprises mixing a compound of formula (I), as hereinbefore defined, with a pharmaceutically acceptable adjuvant, diluent or carrier.

The pharmaceutical compositions may be administered topically (e.g. to the lung and/or airways or to the skin) in the form of solutions, suspensions, heptafluoroalkane aerosols and dry powder formulations; or systemically, e.g. by oral administration in the form of tablets, capsules, syrups, powders or granules, or by parenteral administration in the form of solutions or suspensions, or by subcutaneous administration or by rectal administration in the form of suppositories or transdermally. Preferably the compound of the invention is administered orally.

The invention further relates to combination therapies wherein a compound of formula (1) or a pharmaceutical composition or formulation comprising a compound of formula (1) is administered concurrently or sequentially with therapy and/or an agent for the treatment of any one of asthma, allergic rhinitis, cancer, COPD, rheumatoid arthritis, psoriasis, inflammatory bowel disease, irritable bowel syndrome, osteoarthritis or osteoporosis.

In particular, for the treatment of the inflammatory diseases rheumatoid arthritis, psoriasis, inflammatory bowel disease, irritable bowel syndrome, COPD, asthma and allergic rhinitis the compounds of the invention may be combined with agents such as TNF-α inhibitors such as anti-TNF monoclonal antibodies (such as Remicade, CDP-870 and D.sub2.E.sub7.) and TNF receptor immunoglobulin molecules (such as Enbrel.reg,), non-selective COX-1, / COX-2 inhibitors (such as piroxicam, diclofenac, propionic acids such as naproxen, flubiprofen, fenoprofen, ketoprofen and ibuprofen, fenamates such as mefenamic acid, indomethacin, sulindac, apazone, pyrazolones such as phenylbutazone, salicylates such as aspirin), COX-2 inhibitors (such as meloxicam, celecoxib, rofecoxib, valdecoxib and etoricoxib) low dose methotrexate, lefunomide; ciclesonide; hydroxychloroquine, d-penicillamine, auranofin or parenteral or oral gold. For inflammatory bowel disease and irritable bowel disorder further convenient agents include sulphasalazine and 5-ASAs, topical and systemic steroids, immunomodulators and immunosuppressants, antibiotics, probiotics and anti-integrins.

The present invention still further relates to the combination of a compound of the invention together with a leukotriene biosynthesis inhibitor, 5-lipoxygenase (5-LO) inhibitor or 5-lipoxygenase activating protein (FLAP) antagonist such as zileuton; ABT-761; fenleuton; tepoxalin; Abbott-79175; Abbott-85761; N-(5-substituted)-thiophene-2-alkylsulfonamides; 2,6-di-tert-butylphenol hydrazones; methoxytetrahydropyrans such as Zeneca ZD-2138; the compound SB-210661; pyridinyl-substituted 2-cyanonaphthalene compounds such as L-739,010; 2-cyanoquinoline compounds such as L-746,530; indole and quinoline compounds such as MK-591, MK-886, and BAY x 1005.

The present invention still further relates to the combination of a compound of the invention together with a receptor antagonist for leukotrienes LTB.sub4., LTC.sub4., LTD.sub4., and LTE.sub4. selected from the group consisting of the phenothiazin-3-ones such as L-651,392; amidino compounds such as CGS-25019c; benzoxalamines such as ontazolast; benzenecarboximidamides such as BIIL 284/260; and compounds such as zafirlukast, ablukast, montelukast, pranlukast, verlukast (MK-679), RG-12525, Ro-245913, iralukast (CGP 45715A), and BAY x 7195.

The present invention still further relates to the combination of a compound of the invention together with a PDE4 inhibitor including inhibitors of the isoform PDE4D.

The present invention still further relates to the combination of a compound of the invention together with a antihistaminic H.sub1. receptor antagonists such as cetirizine, loratadine, desloratadine, fexofenadine, astemizole, azelastine, and chlorpheniramine.

The present invention still further relates to the combination of a compound of the invention together with a gastroprotective H.sub2. receptor antagonist.

The present invention still further relates to the combination of a compound of the invention together with an α.sub1.- and α.sub2.-adrenoceptor agonist vasoconstrictor sympathomimetic agent, such as propylhexedrine, phenylephrine, phenylpropanolamine, pseudoephedrine, naphazoline hydrochloride, oxymetazoline hydrochloride, tetrahydrozoline hydrochloride, xylometazoline hydrochloride, and ethylnorepinephrine hydrochloride.

The present invention still further relates to the combination of a compound of the invention together with anticholinergic agents such as ipratropium bromide; tiotropium bromide; oxitropium bromide; pirenzepine; and telenzepine.

The present invention still further relates to the combination of a compound of the invention together with a β.sub1.- to β.sub4.-adrenoceptor agonists such as metaproterenol, isoproterenol, isoprenaline, albuterol, salbutamol, formoterol, sahneterol, terbutaline, orciprenaline, bitolterol mesylate, and pirbuterol; or methylxanthanines including theophylline and aminophylline; sodium cromoglycate; or muscarinic receptor (Ml, M2, and M3) antagonist.

The present invention still further relates to the combination of a compound of the invention together with an insulin-like growth factor type I (IGF-1) mimetic.

The present invention still further relates to the combination of a compound of the invention together with an inhaled glucocorticoid with reduced systemic side effects, such as prednisone, prednisolone, flunisolide, triamcinolone acetonide, beclomethasone dipropionate, budesonide, fluticasone propionate, and mometasone furoate.

The present invention still further relates to the combination of a compound of the invention together with an inhibitor of matrix metalloproteases (MMPs), i.e., the stromelysins, the collagenases, and the gelatinases, as well as aggrecanase; especially collagenase-1 (MMP-1), collagenase-2 (MMP-8), collagenase-3 (MMP-13), stromelysin-1 (MMP-3), stromelysin-2 (MMP-10), and stromelysin-3 (MMP-11) and MMP-12.

The present invention still further relates to the combination of a compound of the invention together with other modulators of chemokine receptor function such as CCR1, CCR2, CCR2A, CCR2B, CCR3, CCR4, CCR5, CCR6, CCR7, CCR8, CCR9, CCR10 and CCR11 (for the C-C family); CXCR1, CXCR3, CXCR4 and CXCR5 (for the C-X-C family) and CX₃CR1 for the C-X₃-C family.

The present invention still further relates to the combination of a compound of the invention together with antiviral agents such as Viracept, AZT, aciclovir and famciclovir, and antisepsis compounds such as Valant.

The present invention still further relates to the combination of a compound of the invention together with cardiovascular agents such as calcium channel blockers, lipid lowering agents such as statins, fibrates, beta-blockers, Ace inhibitors, Angiotensin-2 receptor antagonists and platelet aggregation inhibitors.

The present invention still further relates to the combination of a compound of the invention together with CNS agents such as antidepressants (such as sertraline), anti-Parkinsonian drugs (such as deprenyl, L-dopa, Requip, Mirapex, MAOB inhibitors such as selegine and rasagiline, comP inhibitors such as Tasmar, A-2 inhibitors, dopamine reuptake inhibitors, NMDA antagonists, Nicotine agonists, Dopamine agonists and inhibitors of neuronal nitric oxide synthase), and anti-Alzheimer's drugs such as donepezil, tacrine, COX-2 inhibitors, propentofylline or metryfonate.

The present invention still further relates to the combination of a compound of the invention together with (i) tryptase inhibitors; (ii) platelet activating factor (PAF) antagonists; (iii) interleukin converting enzyme (ICE) inhibitors; (iv) IMPDH inhibitors; (v) adhesion molecule inhibitors including VLA-4 antagonists; (vi) cathepsins; (vii) MAP kinase inhibitors; (viii) glucose-6 phosphate dehydrogenase inhibitors; (ix) kinin-B.sub1.- and B.sub2. -receptor antagonists; (x) anti-gout agents, e.g., colchicine; (xi) xanthine oxidase inhibitors, e.g., allopurinol; (xii) uricosuric agents, e.g., probenecid, sulfinpyrazone, and benzbromarone; (xiii) growth hormone secretagogues; (xiv) transforming growth factor (TGFβ); (xv) platelet-derived growth factor (PDGF); (xvi) fibroblast growth factor, e.g., basic fibroblast growth factor (bFGF); (xvii) granulocyte macrophage colony stimulating factor (GM-CSF); (xviii) capsaicin cream; (xix) Tachykinin NK.sub1. and NK.sub3. receptor antagonists selected from the group consisting of NKP-608C; SB-233412 (talnetant); and D-4418; (xx) elastase inhibitors selected from the group consisting of UT-77 and ZD-0892; (xxi) TNFδ converting enzyme inhibitors (TACE); (xxii) induced nitric oxide synthase inhibitors (iNOS) or (xxiii) chemoattractant receptor-homologous molecule expressed on TH2 cells, (CRTH2 antagonists).

The compounds of the present invention may also be used in combination with osteoporosis agents such as roloxifene, droloxifene, lasofoxifene or fosomax and immunosuppressant agents such as FK-506, rapamycin, cyclosporine, azathioprine, and methotrexate;.

The compounds of the invention may also be used in combination with existing therapeutic agents for the treatment of osteoarthritis. Suitable agents to be used in combination include standard non-steroidal anti-inflammatory agents (hereinafter NSAID's) such as piroxicam, diclofenac, propionic acids such as naproxen, flubiprofen, fenoprofen, ketoprofen and ibuprofen, fenamates such as mefenamic acid, indomethacin, sulindac, apazone, pyrazolones such as phenylbutazone, salicylates such as aspirin, COX-2 inhibitors such as celecoxib, valdecoxib, rofecoxib and etoricoxib, analgesics and intraarticular therapies such as corticosteroids and hyaluronic acids such as hyalgan and synvisc and P2X7 receptor antagonists.

The compounds of the invention can also be used in combination with existing therapeutic agents for the treatment of cancer. Suitable agents to be used in combination include:
(i) antiproliferative/antineoplastic drugs and combinations thereof, as used in medical oncology, such as alkylating agents (for example cis-platin, carboplatin, cyclophosphamide, nitrogen mustard, melphalan, chlorambucil, busulphan and nitrosoureas); antimetabolites (for example antifolates such as fluoropyrimidines like 5-fluorouracil and tegafur, raltitrexed, methotrexate, cytosine arabinoside, hydroxyurea, gemcitabine and paclitaxel (Taxol®); antitumour antibiotics (for example anthracyclines like adriamycin, bleomycin, doxorubicin, daunomycin, epirubicin, idarubicin, mitomycin-C, dactinomycin and mithramycin); antimitotic agents (for example vinca alkaloids like vincristine, vinblastine, vindesine and vinorelbine and taxoids like taxol and taxotere); and topoisomerase inhibitors (for example epipodophyllotoxins like etoposide and teniposide, amsacrine, topotecan and camptothecin);
(ii) cytostatic agents such as antioestrogens (for example tamoxifen, toremifene, raloxifene, droloxifene and iodoxyfene), oestrogen receptor down regulators (for example fulvestrant), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrozole, vorazole and exemestane) and inhibitors of 5α-reductase such as finasteride;
(iii) Agents which inhibit cancer cell invasion (for example metalloproteinase inhibitors like marimastat and inhibitors of urokinase plasminogen activator receptor function);
(iv) inhibitors of growth factor function, for example such inhibitors include growth factor antibodies, growth factor receptor antibodies (for example the anti-erbb2 antibody trastuzumab [Herceptin™] and the anti-erbb1 antibody cetuximab [C225]) , farnesyl transferase inhibitors, tyrosine kinase inhibitors and serine/threonine kinase inhibitors, for example inhibitors of the epidermal growth factor family (for example EGFR family tyrosine kinase inhibitors such as N-(3-chloro-4-fluorophenyl)-7-methoxy-6-(3-morpholinopropoxy)quinazolin-4-amine (gefitinib, AZD1839), N-(3-ethynylphenyl)-6,7-bis(2-methoxyethoxy)quinazolin-4-amine (erlotinib, OSI-774) and 6-acrylamido-N-(3-chloro-4-fluorophenyl)-7-(3-morpholinopropoxy)quinazolin-4-amine (CI 1033)), for example inhibitors of the platelet-derived growth factor family and for example inhibitors of the hepatocyte growth factor family;
(v) antiangiogenic agents such as those which inhibit the effects of vascular endothelial growth factor, (for example the anti-vascular endothelial cell growth factor antibody bevacizumab [Avastin™], compounds such as those disclosed in International Patent Applications WO 97/22596, WO 97/30035, WO 97/32856 and WO 98/13354) and compounds that work by other mechanisms (for example linomide, inhibitors of integrin αvβ3 function and angiostatin);
(vi) vascular damaging agents such as Combretastatin A4 and compounds disclosed in International Patent Applications WO 99/02166, WO00/40529, WO 00/41669, WO01/92224, WO02/04434 and WO02/08213
(vii) antisense therapies, for example those which are directed to the targets listed above, such as ISIS 2503, an anti-ras antisense;
(viii) gene therapy approaches, including for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy; and
(ix) immunotherapy approaches, including for example ex-vivo and in-vivo approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies.

The invention will now be further illustrated by reference to the following examples. In the examples the Nuclear Magnetic Resonance (NMR) spectra were measured on a Varian Unity Inova 300 or 400 MHz spectrometer and the Mass Spectrometry (MS) spectra measured on an Agilent MSD spectrometer Where necessary, the reactions were performed under an inert atmosphere of either nitrogen. Chromatography was generally performed using Matrex Silica 60^{®} (35-70 micron) or Prolabo Silica gel 60^{®} (35-70 micron) suitable for flash silica gel chromatography. High performance liquid chromatography purification was performed using a Gilson Auto-Purification System. The abbreviations m.p. and DMSO used in the examples stand for melting point and dimethyl sulphoxide respectively. Compounds were named using ACD/labs 6.0 naming programme.

### Example 1

### 5-[((2,3-difluorophenyl)methyl)thio]-7-{[(1S,2S)-2-hydroxy-1-(hydroxymethyl)propyl]amino}thiazolo[4,5-d]pyrimidin-2(3H)-one

### a) 6-Amino-2-[[(2,3-difluorophenyl)methyl]thio]-4(3H)-pyrimidinone

4-Amino-6-hydroxy-2-mercaptopyrimidine monohydrate (7.1g) was added portion wise to a stirred suspension of 60% sodium hydride (2.4g) in dry *N,N-*dimethylformamide (70ml). After 1 hour a solution of 2,3-Difluorobenzyl bromide (10g) in dry *N,N*-dimethylformamide (10ml) was added. The solution was stirred over weekend at room temperature, poured on to ice/water and the precipitate was collected by filtration to give 9.6g of the subtitle compound. MS (APCI) (+ve) 270 (M+H, 94%)

### b) 4-Amino-2-[[(2,3-difluorophenyl)methyl]thio]-1,6-dihydro-6-oxo-5-pyrimidinyl ester thiocyanic acid

A mixture of the product from step (a) (28g) and potassium thiocyanate (40.5g) in *N,N-*dimethylformamide (583ml) was heated to 65°C, pyridine (14.5ml) was added and the solution cooled to 5°C. Bromine (5.0ml) was added slowly and the reaction mixture stirred for 2 hours at 5-10°C. poured onto ice water (4200ml), stirred for 1 hour and a solid collected by filtration, washed with water and ether, to give 24g of the subtitle compound.
MS (APCI) (+ve) 327 (M+H)

### c) 2-Amino-5-[[(2,3-difluorophenyl)methyl]thio]-thiazolo[4,5-d]pyrimidin-7(6H)-one

A mixture of the product from step (b) (12.1g), *N,N*-dimethylformamide (70ml) and water (20ml) was heated to 120°C for 24 hours. A colourless solid precipitated from the solution. The mixture was allowed to cool and asolid collected by filtration to give 8.3g of the subtitle compound.
MS (APCI) (+ve) 327 (M+H)

### d) 7-Chloro-5-[[(2,3-difluorophenyl)methyl]thio]-thiazolo[4,5-d]pyrimidin-2-amine

The product of step (c) (10.0g) was suspended in phosphoryl chloride (55ml). N,N-dimethylaniline (5.5ml) was added slowly and reaction mixture heated at reflux for 2 hours. The mixture was allowed to cool, poured onto ice with vigorous stirring ensuring the temperature was not allowed to go above 45°C. After approximately 20 minutes the temperature stabilized at 30°C. The solid that formed was collected by filtration and washed with water. Purified by column chromatography (EtOAc to 5% MeOH in EtOAc) gave 3.34g of the subtitle compound.
MS: APCI (+ve) 345 (M+H)

### e) 2-({2-Amino-5-[((2,3-difluorophenyl)methyl)thio]thiazolo[4,5-d]pyrimidin-7-yl}amino)-(2S,3S)-butane-1,3-diol

The product from step (d) (4g) was suspended in NMP (10ml), triethylamine (2.5ml) and (2*S*,3*S*)-2-aminobutane-1,3-diol (3g) were added. The mixture was heated to 120°C under N₂ for 36 hrs, poured into water (400ml) and ethyl acetate added. The organic phase was separated and washed with saturated brine solution. Evaporation of the solvent left a residue which on trituration with ether:isohexane mixture gave 4.7g of the subtitle compound.
MS: APCI (+ve) 414 (M+H)

### f) 2-({2-Chloro-5-[((2,3-difluorophenyl)methyl)thio]-thiazolo[4,5-d]pyrimidin-7-yl}amino)-(2S,3S)-butane-1,3-diol

The product from step (e) (4.7g) was suspended in conc.HCl (50ml), cooled to 15°C. and a mixture of water (20ml) and acetonitrile (20ml) added. The solution as cooled to 5°C and a solution of sodium nitrite (1.4g) in water (5ml) added dropwise. Then solution was stirred at 5°C for several hours then allowed to warm overnight, cooled to -10°C and neutralized with ammonia and concentrated in vacuo. The precipitate was collected by filtration and washed with water, dried in vacuo to give 3.78g of the subtitle compound.
MS: APCI (+ve) 433/435 (M+H)

### g) 5-[((2,3-difluorophenyl)methyl)thiol-7-{[(1S,2S)-2-hydroxy-1-(hydroxymethyl)propyl]amino}thiazolo[4,5-d]pyrimidin-2(3H)-one

The product from step (f) (1.0g) was suspended in methanol (100ml). Potassium hydroxide (0.4g) was added and mixture stirred at 50°C for 3 hours, neutralised with 2N HCl and solvents removed in vacuo. The orange residue was suspended in a mixture of dioxan (50ml) and conc.HCl (1ml). Water (1ml) was added, the resultant solution was heated at 60°C for 12 hours and cooled to room temperature. Solvents were removed in vacuo and residue triturated with water to give a solid which was collected by filtration and washed with water. Purification using prep. HPLC using Acetonitrile:0.2% ammonium hydroxide as eluent gave 0.35g of the title compound.
mp 202-204°C
MS: APCI (+ve) 415 (M+H)
¹H NMR: δ (DMSO) 1.0 (3H, d), 3.43-3.49 (1H, m), 3.55-3.60 (1H, m), 3.82-3.92 (1H, m), 4.15 (1H, brs), 4.39 (2H, s), 4.43-4.62 (2H, m), 7.08-7.17 (2H, m), 7.29-7.42 (2H, m), 12.41 (1H, s).
CHN C16H16F2N4O3S2 requires C 46.37%, H 3.89%, N 13.52%, S 15.47%; found C 46.26%, H 3.92%, N 13.50%, S 15.44%

### Pharmacological Data

### Ligand Binding Assay

[¹²⁵I]IL-8 (human, recombinant) was purchased from Amersham, U.K. with a specific activity of 2,000Ci/mmol. All other chemicals were of analytical grade. High levels of hrCXCR2 were expressed in HEK 293 cells (human embryo kidney 293 cells ECACC No. 85120602) (Lee et al. (1992) J. Biol. Chem. 267 pp16283-16291), hrCXCR2 cDNA was amplified and cloned from human neutrophil mRNA. The DNA was cloned into PCRScript (Stratagene) and clones were identified using DNA. The coding sequence was sub-cloned into the eukaryotic expression vector RcCMV (Invitrogen). Plasmid DNA was prepared using Quiagen Megaprep 2500 and transfected into HEK 293 cells using Lipofectamine reagent (Gibco BRL). Cells of the highest expressing clone were harvested in phosphate-buffered saline containing 0.2%(w/v) ethylenediaminetetraacetic acid (EDTA) and centrifuged (200g, 5min.). The cell pellet was resuspended in ice cold homogenisation buffer [10mM HEPES (pH 7.4), 1mM dithiothreitol, 1mM EDTA and a panel of protease inhibitors (1mM phenyl methyl sulphonyl fluoride, 2µg/ml soybean trypsin inhibitor, 3mM benzamidine, 0.5µg/ml leupeptin and 100µg/ml bacitracin)] and the cells left to swell for 10 minutes. The cell preparation was disrupted using a hand held glass mortar/PTFE pestle homogeniser and cell membranes harvested by centrifugation (45 minutes, 100,000g, 4°C). The membrane preparation was stored at -70°C in homogenisation buffer supplemented with Tyrode's salt solution (137mM NaCl, 2.7mM KCl, 0.4mM NaH₂PO₄), 0.1%(w/v) gelatin and 10%(v/v) glycerol.
All assays were performed in a 96-well MultiScreen 0.45µm filtration plates (Millipore, U.K.). Each assay contained ~50pM [¹²⁵I]IL-8 and membranes (equivalent to ~200,000 cells) in assay buffer [Tyrode's salt solution supplemented with 10mM HEPES (pH 7.4); 1.8mM CaCl₂, 1mM MgCl₂, 0.125mg/ml bacitracin and 0.1%(w/v) gelatin]. In addition, a compound of formula (I) according to the Examples was pre-dissolved in DMSO and added to reach a final concentration of 1%(v/v) DMSO. The assay was initiated with the addition of membranes and after 1.5 hours at room temperature the membranes were harvested by filtration using a Millipore MultiScreen vacuum manifold and washed twice with assay buffer (without bacitracin). The backing plate was removed from the MultiScreen plate assembly, the filters dried at room temperature, punched out and then counted on a Cobra γ-counter.
The compound of formula (I) has an IC₅₀ value of less than (<) 10µM.

### Intracellular Calcium Mobilisation Assay

Human neutrophils were prepared from EDTA-treated peripheral blood, as previously described (Baly et al. (1997) Methods in Enzymology 287 pp70-72), in storage buffer [Tyrode's salt solution (137mM NaCl, 2.7mM KCl, 0.4mM NaH₂PO₄) supplemented with 5.7mM glucose and 10mM HEPES (pH 7.4)].
The chemokine GROα (human, recombinant) was purchased from R&D Systems (Abingdon, U.K.). All other chemicals were of analytical grade. Changes in intracellular free calcium were measured fluorometrically by loading neutrophils with the calcium sensitive fluorescent dye, fluo-3, as described previously (Merritt et al. (1990) Biochem. J. 269, pp513-519). Cells were loaded for 1 hour at 37°C in loading buffer (storage buffer with 0.1 %(w/v) gelatin) containing 5µM fluo-3 AM ester, washed with loading buffer and then resuspended in Tyrode's salt solution supplemented with 5.7mM glucose, 0.1%(w/v) bovine serum albumin (BSA), 1.8mM CaCl₂ and 1mM MgCl₂. The cells were pipetted into black walled, clear bottom, 96 well micro plates (Costar, Boston, U.S.A.) and centrifuged (200g, 5 minutes, room temperature).

A compound of formula (I) according to the Examples was pre-dissolved in DMSO and added to a final concentration of 0.1 %(v/v) DMSO. Assays were initiated by the addition of an A₅₀ concentration of GROα and the transient increase in fluo-3 fluorescence (λ_{Ex} =490nm and λ_{Em} = 520nm) monitored using a FLIPR (Fluorometric Imaging Plate Reader, Molecular Devices, Sunnyvale, U.S.A.).
The compound of formula (I) was tested and found to be an antagonist of the CXCR2 receptor in human neutrophils.

## Claims

1. A compound of formula (I)

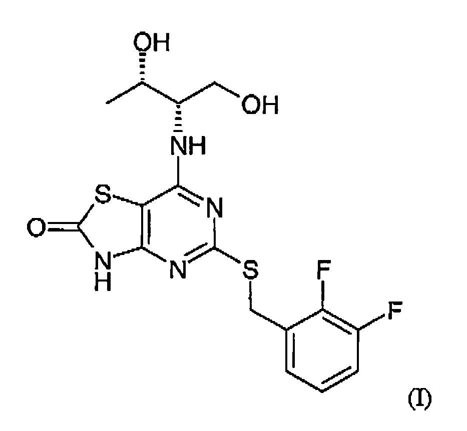

2. A compound of formula (I) as claimed in claim 1 for use in oral therapy.

3. A compound according to claim 1 for use as an oral medicament for the treatment of asthma, allergic rhinitis, COPD, inflammatory bowel disease, osteoarthritis, osteoporosis, rheumatoid arthritis, or psoriasis.

4. A compound according to claim 1 for use as an oral medicament for the treatment of cancer.

5. Use of a compound of formula (I) as claimed in claim 1 in the manufacture of a medicament for use in oral therapy.

6. A compound according to claim 1 for use as an oral medicament in the treatment of an inflammatory disease in a patient suffering from, or at risk of, said disease.

7. A compound as claimed in claim 1 for use in the treatment of a disease in which angiogenesis is associated with raised CXCR2 chemokine levels.

8. A process for the preparation of a compound of formula (I) which comprises reaction of a compound of formula (II): 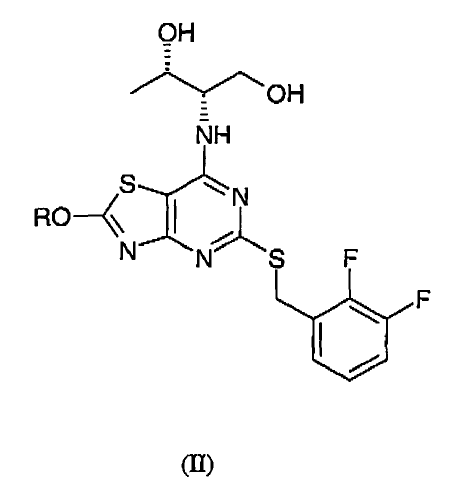 where R is C₁₋₆ alkyl with an acid.

9. Use of a compound according to claim 1 in the manufacture of a medicament for use in. A combination therapy which comprises administering orally a compound of formula (1) or a pharmcaceutical composition or formulation comprising a compound of formula (1), concurrently or sequentially with other therapy and/or another pharmaceutical agent.

10. Use as claimed in claim 9 for the oral treatment of asthma, allergic rhinitis, COPD, inflammatory bowel disease, irritable bowel syndrome, osteoarthritis, osteoporosis, rheumatoid arthritis, or psoriasis.

11. Use as claimed in claim 9 for the oral treatment of cancer.

12. A pharmaceutical composition comprising a compound of formula (I) as claimed in claim 1 in association with a pharmaceutically acceptable adjuvant, diluent or carrier.

13. A pharmaceutical composition as claimed in claim 12 for the oral treatment of asthma, allergic rhinitis, COPD, inflammatory bowel disease, irritable bowel syndrome, osteoarthritis, osteoporosis, rheumatoid arthritis, or psoriasis.

14. A pharmaceutical composition as claimed in claim 12 for the oral treatment of cancer.

15. A process for the preparation of a pharmaceutical composition as claimed in claim 12 which comprises mixing a compound of formula (I) as claimed in claim 1, with a pharmaceutically acceptable adjuvant, diluent or carrier.

## Patentansprüche

1. Verbindung der Formel (I)

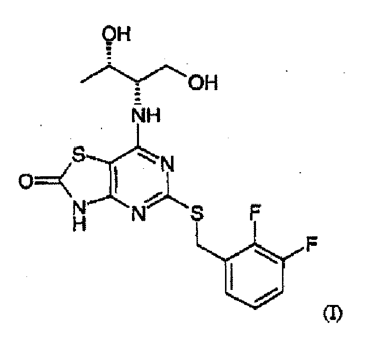

2. Verbindung der Formel (I) nach Anspruch 1 zur Verwendung bei der oralen Therapie.

3. Verbindung nach Anspruch 1 zur Verwendung als orales Arzneimittel zur Behandlung von Asthma, allergischer Rhinititis, COPD, entzündlicher Darmerkrankung, Osteoarthritis, Oesteoporose, rheumatoider Arthritis oder Psoriasis.

4. Verbindung nach Anspruch 1 zur Verwendung als orales Arzneimittel zur Behandlung von Krebs.

5. Verwendung einer Verbindung der Formel (I) nach Anspruch 1 bei der Herstellung eines Arzneimittels zur Verwendung bei der oralen Therapie.

6. Verbindung nach Anspruch 1 zur Verwendung als orales Arzneimittel bei der Behandlung einer entzündlichen Krankheit bei einem Patienten, der an dieser Krankheit leidet oder bei dem das Risiko dieser Krankheit besteht.

7. Verbindung nach Anspruch 1 zur Verwendung bei der Behandlung einer Krankheit, bei der die Angiogenese mit erhöhten CXCR2-Chemokin-Spiegeln assoziiert ist.

8. Verfahren zur Herstellung einer Verbindung der Formel (I), bei dem man eine Verbindung der Formel (II): 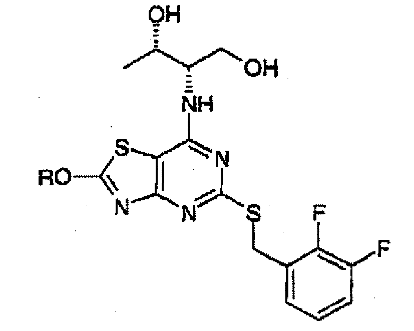 worin R für C₁₋₆-Alkyl steht, mit einer Säure umsetzt.

9. Verwendung einer Verbindung nach Anspruch 1 bei der Herstellung eines Arzneimittels zur Verwendung bei einer Kombinationstherapie, bei der man eine Verbindung der Formel (I) oder eine pharmazeutische Zusammensetzung oder Formulierung, die eine Verbindung der Formel (I) enthält, gleichzeitig oder sequentiell mit einer anderen Therapie und/oder einem anderen Pharmazeutikum oral verabreicht.

10. Verwendung nach Anspruch 9 zur oralen Behandlung von Asthma, allergischer Rhinititis, COPD, entzündlicher Darmerkrankung, Reizkolon, Osteoarthritis, Oesteoporose, rheumatoider Arthritis oder Psoriasis.

11. Verwendung nach Anspruch 9 zur oralen Behandlung von Krebs.

12. Pharmazeutische Zusammensetzung, enthaltend eine Verbindung der Formel (I) nach Anspruch 1 zusammen mit einem pharmazeutisch annehmbaren Hilfsstoff, Verdünnungsmittel oder Träger.

13. Pharmazeutische Zusammensetzung nach Anspruch 12 zur oralen Behandlung von Asthma, allergischer Rhinititis, COPD, entzündlicher Darmerkrankung, Reizkolon, Osteoarthritis, Oesteoporose, rheumatoider Arthritis oder Psoriasis.

14. Pharmazeutische Zusammensetzung nach Anspruch 12 zur oralen Behandlung von Krebs.

15. Verfahren zur Herstellung einer pharmazeutischen Zusammensetzung nach Anspruch 12, bei dem man eine Verbindung der Formel (I) nach Anspruch 1 mit einem pharmazeutisch annehmbaren Hilfsstoff, Verdünnungsmittel oder Träger vermischt.

## Revendications

1. Composé de formule (I)

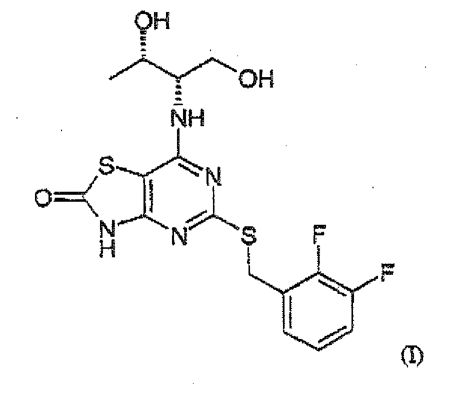

2. Composé de formule (I) selon la revendication 1, destiné à être utilisé en thérapie par voie orale.

3. Composé selon la revendication 1, destiné à être utilisé en tant que médicament oral destiné au traitement de l'asthme, de la rhinite allergique, de la BPCO, de la maladie inflammatoire de l'intestin, de l'ostéoarthrite, de l'ostéoporose, de la polyarthrite rhumatoïde ou du psoriasis.

4. Composé selon la revendication 1, destiné à être utilisé comme médicament oral pour le traitement du cancer.

5. Utilisation d'un composé de formule (I) selon la revendication 1, dans la fabrication d'un médicament destiné à être utilisé en thérapie par voie orale.

6. Composé selon la revendication 1, destiné à être utilisé comme médicament oral dans le traitement d'une maladie inflammatoire chez un patient atteint ou à risque d'être atteint de ladite maladie.

7. Composé selon la revendication 1, destiné à être utilisé dans le traitement d'une maladie dans laquelle l'angiogénèse est associée à des taux accrus de chémokine CXCR2.

8. Procédé de préparation d'un composé de formule (I) qui comprend la réaction d'un composé de formule (II) : 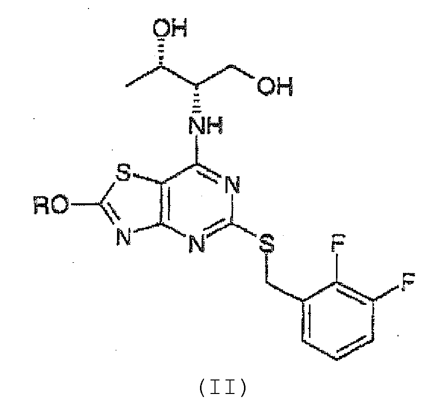 dans laquelle R est alkyle en C₁₋₆ avec un acide.

9. Utilisation d'un composé selon la revendication 1, dans la fabrication d'un médicament destiné à être utilisé dans une thérapie combinée qui comprend l'administration par voie orale d'un composé de formule (I) ou une composition ou une formulation pharmaceutique comprenant un composé de formule (I), simultanément ou séquentiellement avec une autre thérapie et/ou un autre agent pharmaceutique.

10. Utilisation selon la revendication 9, pour le traitement par voie orale de l'asthme, de la rhinite allergique, de la BPCO, de la maladie inflammatoire de l'intestin, du syndrome du côlon irritable, de l'ostéoarthrite, de l'ostéoporose, de la polyarthrite rhumatoïde ou du psoriasis.

11. Utilisation selon la revendication 9, pour le traitement par voie orale du cancer.

12. Composition pharmaceutique comprenant un composé de formule (I) selon la revendication 1, en association avec un adjuvant, un diluant ou un support pharmaceutiquement acceptable.

13. Composition pharmaceutique selon la revendication 12, destinée au traitement par voie orale de l'asthme, de la rhinite allergique, de la BPCO, de la maladie inflammatoire de l'intestin, du syndrome du côlon irritable, de l'ostéoarthrite, de l'ostéoporose, de la polyarthrite rhumatoïde ou du psoriasis.

14. Composition pharmaceutique selon la revendication 12 destinée au traitement par voie orale du cancer.

15. Procédé de préparation d'une composition pharmaceutique selon la revendication 12, **caractérisé en ce qu'**il comprend le mélange d'un composé de formule (I) selon la revendication 1, avec un adjuvant, un diluant ou un support pharmaceutiquement acceptable.
